# EUROPEAN PATENT SPECIFICATION

(11) **EP 1 259 261 B1**
(45) Date of publication and mention of the grant of the patent: **15.06.2005**
(21) Application number: 01902337.3
(22) Date of filing: 18.01.2001
(51) Int. Cl.: A61K 47/02, A61K 47/12, A61K 47/30, A61K 47/36, A61K 9/20, A61K 31/137

(54) **PHARMACEUTICAL COMPOSITIONS COMPRISING TERBINAFINE**
PHARMAZEUTISCHE ZUSAMMENSETZUNGEN ENTHALTEND TERBINAFIN
COMPOSITIONS PHARMACEUTIQUES RENFERMANT DU TERBINAFINE

(30) Priority: 20.01.2000 GB 0001315
(43) Date of publication of application: 27.11.2002
(73) Proprietor: Novartis AG, 4056 Basel (CH); Novartis Pharma GmbH, 1230 Wien (AT)
(72) Inventor: HIRSCH, Stefan, 79539 Lörrach (DE); BECKER, Dieter, 79102 Freiburg (DE); GUITARD, Patrice, F-68220 Hegenheim (FR); EUGSTER, Andreas, Carl, CH-4123 Allschwil (CH)
(74) Representative: Gros, Florent
(86) International application number: PCT/EP2001/000562
(87) International publication number: WO 2001/052895

(56) References cited:
- EP-A- 0 390 369
- WO-A-85/01656
- WO-A-96/35423
- US-A- 4 894 375
- "Rote Liste" 1996 , EDITIO CANTOR , AULENDORFF/WÜRTT. XP002164294 no. 21020 "Lamisil"

## Description

The present invention relates to an oral pharmaceutical composition comprising terbinafine.

Terbinafine is known from e.g. BE-PS-853976 and EP-A-24587. It belongs to the class of allylamine anti-mycotics. It is acknowledged in the art and is commercially available under the trade name Lamisil®. Terbinafine is highly active upon both topical and oral administration. While numerous pharmaceutical compositions for topical and oral administration have been proposed, there still exists a need for commercially acceptable terbinafine formulations for oral administration with good patient convenience and acceptance, especially for children or the elderly. One particular difficulty in the formulation of terbinafine in oral pharmaceutical compositions is its unpleasant, e.g. bitter, taste and/or low physical integrity.

Applicants have now surprisingly found that pharmaceutically acceptable solid dosage forms of terbinafine showing rapid disintegration in aqueous medium, e.g. upon oral administration, and having an acceptable taste can been obtained by formulating buffered pharmaceutical compositions (hereinafter called compositions of the present invention) comprising terbinafine as the active agent and one or more disintegrants. More specifically, it was found that the inclusion of a suitable buffer in a sufficient amount provided taste-masking properties to the compositions of the invention. Furthermore, these rapidly disintegrating compositions of the invention were found to show high stability and physical integrity, e.g. during storage, handling, packaging and the like, without limiting the disintegration performance of the composition.

Accordingly in one aspect the present invention provides a solid pharmaceutical composition for oral administration having a disintegration time of 90 seconds or less in an aqueous medium such as the oral cavity and comprising terbinafine, a buffering component singly or in any suitable combination and one or more disintegrants, wherein the buffering component is capable of maintaining a pH of 5 to 6 on treatment with excess water.

Terbinafine may be used e.g. in free base form or in acid addition salt form. An acid addition salt form may be prepared from the free base form in conventional manner and vice-versa.

Examples of suitable acid addition salt form are the hydrochloride, the lactate and the ascorbate, preferably the hydrochloride.

Terbinafine may be present in an amount of 0.1 to 70 %, e.g. 1 to 60%, preferably 5 to 55% by weight based on the total weight of the composition.

Suitable disintegrants according to the present invention include those pharmaceutical excipients which facilitate the disintegration of a solid dosage form, e.g. a tablet, when placed in an aqueous environment, and comprise the following:
(i) natural starches, such as maize starch, potato starch, and the like, directly compressible starches, e.g. Sta-rx® 1500, modified starches, e.g. carboxymethyl starches and sodium starch glycolate, available as Primojel®, Explotab®, Explosol®, and starch derivatives such as amylose;
(ii) crosslinked polyvinylpyrrolidones, e.g. crospovidones, e.g. Polyplasdone® XL and Kollidon® CL;
(iii) alginic acid and sodium alginate;
(iv) methacrylic acid-divinylbenzene copolymer salts, e.g. Amberlite® IRP-88; and
(v) cross-linked sodium carboxymethylcellulose, available as e.g. Ac-di-sol®, Primellose®, Pharmacel® XL, Explocel®, and Nymcel® ZSX

Preferred disintegrants include those from classes (i) and (ii), particularly preferred are Starx®, Primojel® and Polyplasdone®.

In a further aspect the present invention provides a composition of the present invention wherein the disintegrant is Primojel^{R}, Sta-Rx^{R} and/or Polyplasdone XL^{R}.

The disintegrant may be present in an amount of 1 to 50 %, e.g. 5 to 40% by weight based on the total weight of the composition.

In a further aspect the present invention provides a pharmaceutical composition according to the present invention wherein the ratio of terbinafine : disintegrant may be 1 : 0.1 to 20, e.g. 1 : 0.5 to 15, preferably 1 : 0.5 to 10.

The compositions of the invention comprise a suitable buffering component, e.g. a salt of an acid that is partially dissociated in aqueous solution, include those which - upon disintegration of the composition in an aqueous medium (e.g. the oral cavity) - are capable of maintaining a pH at which terbinafine remains substantially insoluble, e.g. a pH in acidic range, e.g. a pH of greater than 4, preferably a pH of 5 to 6, on treatment with excess water, e.g. 5 to 100 ml. Examples of suitable buffers for use in this invention include carbonate, citrate, acetate, phosphate, phthalate, tartrate salts of the alkali and alkaline earth metal cations, such as sodium, potassium, magnesium and calcium. Preferred buffering agents include e.g. calcium carbonate, trisodium citrate and sodium hydrogen carbonate.

In another aspect the present invention provides a composition of the present invention wherein the buffering component is calcium carbonate, trisodium citrate and/or sodium hydrogen carbonate.

The buffering agents may be used singly or in any suitable combination for achieving the desired pH and may be of a buffer strength of 0.01 to 1 moles/litre, preferably 0.01 to 0.1 moles/litre.

The molar ratio of terbinafine to buffering component in the composition of the present invention may be between 0.02 and 10, e.g. 0.2 and 10, preferably 0.5 to 5, more preferably 0.5 and 2.

The compositions of the invention may further comprise further components which are commonly employed in the preparation of dosage forms, e.g. solid dosage forms. These components include, among others, binders, fillers, lubricants, e.g. magnesium stearate, and glidants, e.g. silica.

In another aspect the present invention provides a composition of the present invention further comprising one or more components selected from binders, fillers, lubricants, and glidants.

Suitable binders according to the present invention include the following:
(i) modified celluloses, e.g. hydroxypropyl methylcellulose (HPMC) available as e.g. Pharmacoat® 603;
(ii) cross-linked sodium carboxymethylcellulose, available as e.g. Ac-di-sol®, Primellose®, Pharmacel® XL, Explocel®, and Nymcel® ZSX; and
(iii) polyvinylpyrrolidone available as e.g. Kollidon® or Plasdone®.
Preferred binders include e.g. Pharmacoat®.

The binder may be present in an amount of 0.5 to 50 %, e.g. 1 to 40%, e.g. 1-25%, e.g. 1 to 15%, preferably 1 to 8% by weight based on the total weight of the composition.

In a further aspect the present invention provides a pharmaceutical composition according to the present invention wherein the ratio of terbinafine : binder may be 1 : 0.01 to 10, e.g. 1 : 0.05 to 5, preferably 1 : 0.1 to 1.

Suitable fillers according to the present invention include excipients known for their properties as filler and plasticizing agents, and include:
(i) substantially water insoluble excipients, such as microcrystalline cellulose (which may also be regarded as a weak disintegrant), e.g. Avicel®, Pharmacel®, Emcocel®, Vivapur®, preferably Avicel®;
(ii) substantially water soluble excipients, such as compression sugars, e.g. lactose,
(iii) sucrose, amylose, dextrose, mannitol, inositol, and the like, preferably lactose; and
(iv) calcium hydrogen orthophosphate dihydrate, e.g. Emcompress®.

If present, the filler may be present in an amount of 0.1 to 50 %, e.g. 1 to 40%, preferably 5-30% by weight based on the total weight of the composition.

In a further aspect the present invention provides a pharmaceutical composition according to the present invention wherein the ratio of terbinafine : filler may be 1 : 0.01 to 100, e.g. 1 : 0.01 to 20, e.g. 1 : 0.01 to 10, preferably 1 : 0.1 to 5.

It will be appreciated that the present invention encompasses
a) in respect of the disintegrant any of components i) to iv) individually or in combination with one or more of the other components i) to iv),
b) in respect of the buffer any of the buffers specified above individually or in combination,
c) in respect of the binder and filler any of those specified above individually or in combination.

The compositions may also include one or more further additives or ingredients in an amount of from e.g. 0.01 to 5 % by weight based on the total weight of the composition, for example sweetening agents, e.g. sorbitol, saccharin, aspartame, acesulfame, or sugars, e.g. glucose, fructose or saccharose; flavouring agents, e.g. chocolate, cocoa, banana, strawberry, or vanilla flavour, and so forth.

In another aspect the composition of the present invention further comprises sweetening and flavouring compounds.

Determination of workable proportions in any particular instance will generally be within the capability of the man skilled on the art. All indicated proportions and relative weight ranges described above are accordingly to be understood as being indicative of preferred or individually inventive teachings only and not as limiting the invention in its broadest aspect.

Details of any of the excipients of the invention may be found in Fiedler, H. P. "Lexikon der Hilfsstoffe für Pharmazie, Kosmetik und angrenzende Gebiete", Editio Cantor Verlag Aulendorf, Aulendorf, 4th revised and expanded edition (1996); "Handbook of Pharmaceutical Excipients", 2nd Edition, Editors A. Wade and P. J. Weller (1994), Joint publication of American Pharmaceutical Association, Washington, USA and The Pharmaceutical Press, London, England; or may be obtained from the relevant manufacturers, the contents of which are hereby incorporated by reference.

In yet another aspect the invention provides a process for the production of a pharmaceutical composition as defined above using conventional methods, e.g. wet granulation methods, roller compaction, dry blending, extrusion/spheronization, melt extrusion, to ensure homogeneous mixing of the components. Conveniently, tablets may be formed from such formulations by conventional methods, e.g. compressing methods. The process is preferably carried out in the absence of water, e.g. only anhydrous solvents are used. Methods for making dry pharmaceutical formulations, e.g. tablets, powder or granules, are well-known and described e.g. in Remington's Pharmaceutical Sciences, 18th Edition, Ed.: Alfonso R. Gennaro, Easton, PA : Mack, 1990, the contents of which are incorporated herein.

The compositions of the present invention thus obtained show fast disintegration in an aqueous medium and have an acceptable taste and thus have particularly good patient convenience and patient acceptance due to their increased ease of administration and ingestion.

Thus, the compositions of the present invention, which are conveniently in solid form, e.g. in the form of a tablet, powder or granule, preferably in the form of a tablet, may be administered as such or, if desired, dissolved prior to administration in a small amount of a liquid, e.g. water, milk or juice, in e.g. a spoon.

In another aspect the present invention provides a composition of the present invention in form of a tablet, powder or granule.

In addition the composition of the present invention shows surprisingly high physical stability, e.g. for up to two or more years. The physical stability may be tested in conventional manner, e.g. the compositions may be tested as such by measurement of dissolution, disintegration time, by hardness test and/or microscopy, e.g. after storage at room temperature, i.e. at 25°C, and/or after storage at 40°C. The taste of the compositions may be tested in standard clinical studies.

The compositions of this invention are useful for the known indications of terbinafine, e.g. for the following conditions: onychomycosis caused by dermatophyte fungi, tinea capitis, fungal infections of the skin, for the treatment of tinea corporis, tinea cruris, tinea pedis, and yeast infections of the skin caused by the genus Candida, e.g. Candida albicans, systemic mycosis, mycosis by azole-resistant strains, e.g. in combination with a 14-alpha-methyldimethylase inhibitor, or infections with Helicobacter pylori.

The composition is particularly effective in treating tinea capitis in e.g. children.

In a further aspect the present invention provides a method for the treatment of fungal infections of the human body comprising administering a pharmaceutically effective amount of a pharmaceutical composition according to the present invention to a subject in need of such treatment.

In yet a further aspect the present invention provides the use of a composition according to the present invention in the manufacture of a medicament for the treatment or fungal infections of the human body.

The utility of the pharmaceutical compositions of the present invention may be observed in standard bioavailability tests or standard animal models, for example ascertaining dosages of the present compositions giving blood levels of terbinafine equivalent to blood levels giving a therapeutical effect on administration of known terbinafine oral dosage forms, e.g. a tablet. Typical doses are in the range of 1 mg/kg to 10 mg/kg, e.g. 1.5 mg/kg to 5 mg/kg, or e.g. 3 to 4 mg/kg body weight of terbinafine per day.

The appropriate dosage will, of course, vary depending upon, for example, the host and the nature and severity of the condition being treated. However in general satisfactory results in animals are indicated to be obtained at daily treatments with doses from about 1 mg/kg to about 10 mg/kg animal body weight. In humans an indicated daily dosage is in the range from about 10 mg to about 1000 mg per day, conveniently administered, for example, in divided doses up to four times a day or once daily. Preferred dosages for children weighing < 20 kg may be about 62.5 mg once daily, for children weighing 20 to 40 kg about 125 mg once daily, for children weighing > 40 kg about 250 mg once daily, and for adults from about 250 mg to about 500 mg once daily.

Following is a description by way of example only of compositions of the invention.

### Example 1 and 2

Tablets according to the invention and of the following composition were prepared by mixing the ingredients at dry stage and compressing the resultant mixture.

| **Components** | **Example 1** | | **Example 2** | |
|---|---|---|---|---|
| | % | mg/tablet | % | mg/tablet |
| Terbinafine HCl granulated with 5% HPMC | 37.1 | 148.05* | - | - |
| Terbinafine HCl granulated with 5% HPMC, Avicel® PH 102, Sta-Rx®-1500 and Cocoa | - | - | 68.1 | 280.0** |
| Sodium hydrogen carbonate | 10 | 40.0 | 9.7 | 40.0 |
| Primojel® | 15.6 | 62.5 | 7.5 | 31.0 |
| Avicel® PH101 | 15.6 | 62.5 | - | - |
| Sta-Rx® 1500 | 15.6 | 62.5 | 9.7 | 40.0 |
| Aspartame (sweetening agent) | 2 | 8.0 | 1.9 | 8.0 |
| Aerosil® 200 | 1 | 4.0 | 1.0 | 4.0 |
| Cocoa (flavouring agent) | 2 | 8.0 | - | - |
| Chocolate (flavouring agent) | 0.5 | 2.0 | 0.5 | 2.0 |
| Magnesium stearate | 0.5 | 2.0 | 1.5 | 6.0 |
| | | | | |
| Total | 99.9 | 399.55 | 99.9 | 411.0 |

| | | | | |
|---|---|---|---|---|
| *corresponds to 141 mg Terbinafine HCl; granulated with water. | | | | |
| **corresponds to 141 mg Terbinafine HCl, 78 mg Avicel® PH 102, 38 mg Sta-Rx® 1500, 8 mg Cocoa and 15 mg HPMC; granulated with water. | | | | |

### Example 3

Tablets according to the invention and of the following composition were prepared by mixing the ingredients at dry stage and compressing the resultant mixture.

| **Components** | **%** | **mg/tablet** |
|---|---|---|
| Terbinafine HCl granulated with 5% HPMC | 36.7 | 148.05* |
| Calcium carbonate | 24.8 | 100.0 |
| Trisodium citrate dihydrate | 8.7 | 35.0 |
| Polyplasdone XL® | 24.8 | 100.0 |
| Aspartame (sweetening agent) | 2 | 8.0 |
| Cocoa (flavouring agent) | 2 | 8.0 |
| Chocolate (flavouring agent) | 0.5 | 2.0 |
| Magnesium stearate | 0.5 | 2.0 |
| | | |
| Total | 100.0 | 403.05 |

| | | |
|---|---|---|
| *corresponds to 141 mg Terbinafine HCl; granulated with water. | | |

### Example 4

Tablets according to the invention and of the following composition were prepared by compression:

| **Components** | **%** | **mg/tablet** |
|---|---|---|
| Terbinafine HCl | 32.5 | 140.625 |
| Primojel® | 5.2 | 22.425 |
| Avicel® PH 101 | 5.6 | 24.15 |
| Pharmacoat® 603 (HPMC) | 1.4 | 5.85 |
| Magnesium stearate | 0.8 | 3.215 |
| Aerosil® 200 | 0.2 | 0.975 |
| Calcium Carbonate (Destab 90) | 23.1 | 100.00 |
| Polyplasdon® XL | 23.1 | 100.00 |
| Trisodium citrate dihydrate | 8.1 | 35.0 |
| | | |
| Total | 100.0 | 432.24 |

Document D1 (US-A-4'894'375) discloses water-dispersible tablets containing pharmaceutically active substances as microparticles and comprising a disintegrant and swellable material able to generate a high viscosity on contact with water, and disintegrating rapidly in water to form a homogeneous suspension of high viscosity which can easily be swallowed. There is no mention of buffers nor taste-masking.

Document D2 (WO 96/35423) provides compositions active against azole-resistant fungal strains. It does not mention taste-masking and there is no suggestion to use a buffering component.

Document D3 ("Rote Liste" 1996, Editio Cantor, Aulendorff/Württ, XP 002164294) discloses terbinafine tablets and their composition. While there is mention of rare and transient taste disturbances normally reversible within a few weeks, there is no mention of unpleasant taste of terbinafine oral compositions, nor suggestion to use a buffering component.

## Claims

1. A solid pharmaceutical composition for oral administration having a disintegration time of 90 seconds or less in an aqueous medium such as the oral cavity and comprising terbinafine, a buffering component singly or in any suitable combination and one or more disintegrants, wherein the buffering component is capable of maintaining a pH of 5 to 6 on treatment with excess water.

2. A composition according to claim 1 further comprising one or more components selected from binders, fillers, lubricants, and glidants.

3. A composition according to any preceding claim further comprising sweetening and flavouring compounds.

4. A composition according to any preceding claim in form of a tablet, powder or granule.

5. A composition according to any preceding claim wherein the buffering component is calcium carbonate, trisodium citrate and/or sodium hydrogen carbonate.

6. A composition according to any preceding claim wherein the disintegrant is Primojel^{R}, Sta-Rx^{R} and/or Polyplasdone XL^{R}.

7. Use of a composition according to claim 1 in the manufacture of a medicament for the treatment of fungal infections of the human body.

## Patentansprüche

1. Feste pharmazeutische Zusammensetzung zur oralen Verabreichung mit einer Zerfallzeit von 90 s oder weniger in einem wässrigen Medium, wie der Mundhöhle, und einem Gehalt an Terbinafin, einer Pufferkomponente einzeln oder in irgendeiner geeigneten Kombination und ein oder mehr Zerfallmitteln, worin die Pufferkomponente zur Aufrechterhaltung eines pH Werts von 5 bis 6 bei Behandlung mit überschüssigem Wasser befähigt ist.

2. Zusammensetzung nach Anspruch 1, die ferner ein oder mehr Komponenten enthält, welche ausgewählt sind aus Bindemitteln, Füllstoffen, Schmiermitteln und Gleitmitteln.

3. Zusammensetzung nach einem der vorhergehenden Ansprüche, die ferner Süßstoffe und Aromastoffe enthält.

4. Zusammensetzung nach einem der vorhergehenden Ansprüche in Form einer Tablette, eines Pulvers oder eines Granulats.

5. Zusammensetzung nach einem der vorhergehenden Ansprüche, worin die Pufferkomponente Calciumcarbonat, Trinatriumcitrat und/oder Natriumhydrogencarbonat ist.

6. Zusammensetzung nach einem der vorhergehenden Ansprüche, worin das Zerfallmittel Primojel®, Sta-Rx® und/oder Polyplasdon XL® ist.

7. Verwendung einer Zusammensetzung nach Anspruch 1 bei der Herstellung eines Arzneimittels für die Behandlung fungaler Infektionen des menschlichen Körpers.

## Revendications

1. Composition pharmaceutique solide destinée à être administrée par voie orale présentant un temps de désintégration de 90 secondes ou moins dans un milieu aqueux tel que la cavité buccale et comprenant de la terbinafine, un composant tampon isolé ou présent dans toute combinaison adéquate ainsi qu'un ou plusieurs délitants, **caractérisée en ce que** le composant tampon est capable de maintenir le pH à une valeur de 5 à 6 lors d'un traitement avec surplus d'eau.

2. Composition selon la revendication 1 comprenant également un ou plusieurs composants sélectionnés parmi les liants, les charges, les lubrifiants et les glissants.

3. Composition selon l'une des revendications précédentes comprenant également des composés édulcorants et aromatiques.

4. Composition selon l'une des revendications précédentes se présentant sous forme d'un comprimé, de poudre ou de granules.

5. Composition selon l'une des revendications précédentes **caractérisée en ce que** le composant tampon est du carbonate de calcium, du citrate trisodique et/ou de l'hydrogénocarbonate de sodium.

6. Composition selon l'une des revendications précédentes **caractérisée en ce que** le délitant est du Primojel®, du Sta-Rx® et/ou du Polyplasdone XL®.

7. Utilisation d'une composition selon la revendication 1 dans la fabrication d'un médicament pour le traitement d'infections fongiques du corps humain.
